# EUROPEAN PATENT SPECIFICATION

(11) **EP 1 827 261 B1**
(45) Date of publication and mention of the grant of the patent: **03.12.2008**
(21) Application number: 05792285.8
(22) Date of filing: 02.09.2005
(51) Int. Cl.: A61B 17/44

(54) **AN OBSTETRICAL VACUUM CUP**
SAUGGLOCKE FÜR DIE GEBURTSHILFE
VENTOUSE OBSTETRIQUE

(30) Priority: 09.09.2004 DK 200401365
(43) Date of publication of application: 05.09.2007
(73) Proprietor: Morch, Jacob Sebastian Lunau, 3300 Vaerlose (DK)
(72) Inventor: Morch, Jacob Sebastian Lunau, 3300 Vaerlose (DK)
(74) Representative: Holme Patent A/S
(86) International application number: PCT/IB2005/052878
(87) International publication number: WO 2006/027731

(56) References cited:
- EP-B- 0 923 348
- FR-E- 65 209
- GB-A- 2 248 223
- US-A- 4 730 617
- US-A- 6 074 399

## Description

The present invention relates to an obstetrical vacuum cup of the kind comprising a base and a side wall defining a hollow interior cavity, said side wall having a distal edge which defines a first contact surface ; the cup further comprising a vacuum opening communicating with the interior cavity of the vacuum cup and being adapted for connection to an external vacuum source.

During childbirth, the birth mother is sometimes unable to deliver the fetus without assistance, for example, in conditions of dystocia (i.e., slow or difficult labour or delivery), uterine inertia, maternal exhaustion, maternal distress, or fetal distress. Such assistance generally entails the use of a device to aid in the delivery of the fetus. These devices may likewise be required to assist a physician during particularly difficult Caesarean sections.

One example of such a device is forceps. Forceps, however, tend to be bulky and difficult to operate. In addition, the use of forceps, at the very least, is uncomfortable for both the mother and the fetus and risks injury to both.

Alternative devices to forceps are obstetrical vacuum extractors. Conventional obstetrical vacuum extractor systems utilize a cup, which may be placed onto the fetal head, an elongated traction device with a handle for applying the traction force, and, traditionally, an elongated tube coupled at one end to the interior of the cup and the other end to a electrical floor based or a hand-held vacuum pump. Operation of the hand-held vacuum pump results in the development of a vacuum between the cup and the fetal head, which is then used to manoeuvre and extract the fetus from the birth canal by pulling in an appropriate direction on the gripping device.

The tube may be coupled directly to the cup or coupled to an elongated hollow stem, which acts as the traction device.

More recently, unitary obstetrical vacuum extractor devices have been developed which include a vacuum extractor cup integrated with a hand-held pump and pulling handle. In this way, once properly placed on the fetal head, the physician can both apply an extraction force and manoeuvre the cup while applying and maintaining a vacuum within the cup, all with the same hand. Such devices are disclosed in, for example U. S. Patent No. 5,395,379 and U. S. Patent No. 6,074,399. From US 4,730,617 is known a vacuum extractor with two vessel chambers, each of which is evacuable seperately.

While vacuum assisted fetal extractors have provided many advantages in the delivery of a fetus from its mother, such devices also pose potential hazards if not properly utilized or adequately monitored during their use. Such hazards stem from the fact that the fetal skull is soft and structurally weak and thus renders the head of the fetus susceptible to deformation or injury during childbirth.

One example that can be mentioned is that a fetal vacuum cup must be applied to the fetal head so that the center of the cup corresponds as close as possible to the flexion point of the fetal head. The positioning of the center of the cup on the flexion point is important to ensure a safe and relatively easy vacuum-assisted delivery. Traction applied along the pelvic axis from a properly positioned vacuum cup promotes proper fetal presentation as the fetus passes through the birth canal.

However, as the fetus often malpresents such that the flexion point is pressed against the birth canal walls, proper positioning of the fetal vacuum cup is difficult because the vacuum cup must be inserted between the birth canal wall and the occiput. Thus, the centers of the fetal vacuum cups are often not properly applied over the flexion point of malpresenting fetuses.

Furthermore, the manoeuvrability of the fetal vacuum cup affects the correct application of the vacuum cup over the flexion point of a malpresenting fetus.

Misapplication or improper use of a vacuum assisted fetal extractor may also result in a number of injuries to the fetus. Such injuries might include, by way of example only, subdural hematoma, subgaleal hemorrhage, abrasions, as well as other, less common injuries. While not an exhaustive list, injuries of the foregoing type listed above may be the result of improper placement of the vacuum cup on the fetal head, application of an excessive vacuum, or maintaining a vacuum for an excessive continuous duration.

Because all vacuum extractor cups, in order to work, depend on a firm attachment to the fetal scalp by suction, all of them cause a swelling of the scalp (called a chignon or iatrogenic caput succedaneum) which is harmless and which may take as little as 2 hours or as long as 2 weeks to resolve. Similarly, some marking of the fetal scalp will normally be present at the site of cup attachment but the extent to which it is visible will depend on the amount of hair present on the fetal scalp. Such markings disappear after a few days without leaving any traces.

These swellings are today associated with a significant incidence of jaundice [Bertini et al., Is Breastfeeding Really Favoring Early Neonatal Jaundice?, Pediatrics. 2001 ;107:3:e41], and it is therefore desirable to provide a cup which reduces or eliminates such swelling.

Soft vacuum extractor cups made of silicone or soft plastic materials appear to cause less obvious marking and swelling of the scalp than the metal or rigid plastic cups but they are less likely to complete the delivery than the metal cups and they detach more readily from the scalp.

Furthermore, although vacuum extractor devices are widely used, the edge of the cup, which forms the seal between the cup and the fetal head, defines a relatively small contact surface through which the forces, which result from operation and manipulation of the extractor, are transmitted to the fetal head. Since the contact surface between the cup and the fetal head is relatively small, the resultant forces caused by operation of the extractor are concentrated over a corresponding relatively small area of the fetal scalp.

Moreover, a small contact surface will result in a number of unintended cup detachments due to the fact that the cup is not applied to the correct site or that traction is not applied perpendicular to the cup with the result that one side of the cup is lifted up. When the cup detaches from the fetal scalp this will happen with a jerk, applying additional stress and discomfort for both mother and child. Furthermore, as the cup detaches it will often slide across the fetal scalp resulting in a number of lacerations.

Thus, it is a first aspect of the present invention to provide a device of the kind mentioned in the opening paragraph, which will reduce the swelling of the scalp during use.

It is a second aspect of the present invention to provide a device of the kind mentioned in the opening paragraph, which will reduce the risk of unintended cup detachments during delivery and/or provide the operator with extra time before the cup detaches.

It is a third aspect of the present invention to provide a device of the kind mentioned in the opening paragraph, which is simple and reliable in design and in operation and capable of being adapted to existing vacuum extraction pumps.

It is a fourth aspect of the present invention to provide a device of the kind mentioned in the opening paragraph, which is conducive to long-term storage and inexpensive to manufacture so as to enable its disposal after a single use.

It is a fifth aspect according to the present invention to provide a filter device capable of reducing or eliminating blood and/or body fluid contamination of the external vacuum source.

This is achieved according to the present invention, as the hollow interior cavity is designed with at least one projection providing at least one second contact surface capable of forming a seal in contact with a fetal head when vacuum is applied from an external source of vacuum and that the cup has at least one vacuum channel which extends from the vacuum opening through the at least one projection, enabeling the vacuum to be applied in the different vacuum compartments of the cup during use.

By this design the forces, resulting from the application of vacuum to the cup and manipulation of the extractor, will be transmitted to the fetus through both the first and the second contact surfaces, distributing the forces over a greater area of the fetal head. Accordingly, the potential for injury to the fetus caused by a concentration of forces is reduced.

The at least one projection will also have the advantage, that the swelling of the scalp, the so-called chignon, will be significantly reduced or even in some instances eliminated. Thereby not only is the risk of jaundice lowered, but the disfigured marks normally associated with children delivered by use of a vacuum extractor will also be reduced.

Depending on the placement of the projections, the interior cavity can be divided into a number of vacuum compartments. Said vacuum compartments will have the advantage, that if the distal edge of the cup detaches from the fetal scalp during extraction only the vacuum compartment(s) adjacent to the distal edge of the cup will loose its grip with the fetal scalp.

The vacuum seal established with the remaining compartments will break one by one with a small time lag between the breaks. This will provide the operator with extra time to e.g. support the fetal head before the cup detaches in full, thereby eliminating the jerks and the lacerations on the scalp normally involved in cup detachments. The cup according to the invention is therefore not only much more attractive for mother and child, it is also a better alternative for the operator as the use of the cup is both safer and easier.

The projections also have the advantage that they constitute a filter device, i.e. they present a physical barrier for e.g. blood or body fluids from mother and/or fetus entering the interior cavity, and are thus reducing the risk of contaminating the vacuum cup device, e.g. the external vacuum pump.

The projections can advantageously be in the form of concentric rings, collars or cylinders arranged perpendicularly on the interior of the base. The projections can e.g. be attached to the interior of the base using adhering means well known for the person skilled in the art, e.g. glue, or the projections can be an integrated part of the base.

Thereby, not only is a simple and effective solution obtained for enabling a larger contact surface with the fetal head, but such projections can also ensure, that if only one vacuum compartment is adjacent to the distal edge only one vacuum compartment is able to detach at a time. Thereby a more controlled cup detachment is provided as compared with conventional extractors.

It is preferred, that the vacuum opening is placed at the center of the base, as this will ensure a unified removal of air from different compartments when vacuum is applied to the cup from the external source of vacuum. This will also allow the forces from the contact surface to be distributed uniformly over the selected area on the fetal head when vacuum is applied.

The projections can advantageously be of an elastomeric material, such as a thermoplastic elastomer, as this will provide a soft and flexible second contact surface with the fetal scalp. Said material preferably has a memory. That is to say, the projections of the device according to the invention will automatically try to return to their original shape when they have been mechanically deformed and then relieved.

It is furthermore desirable that the material toughing the fetal scalp preferably are made of a material, which significantly reduce swellings on the infants scalp and at the same time is capable of providing a vacuum in the interior cavity.

In the same manner the first contact surface can be at least partly covered with a sealing member ensuring a more effective sealing of the interior cavity of the cup. Furthermore, if the sealing member is made of a relatively soft elastomeric material, e.g. a thermoplastic elastomer, a simple and inexpensive design is applied which also provides a soft first contact surface. Thereby is the potential for injury to the maternal and fetal tissue during positioning and delivery reduced.

Preferably, the distal edge of the annular sidewall comprises a structure, which enhances the attachment of the sealing member. While various designs may be appropriate, the vacuum cup may include one or more annular ridges, which extend about the periphery of the distal edge of the cup. The ridges provide additional gripping surfaces for the material of the sealing member.

In another embodiment according to the invention, the sealing member and the projections are made from an elastomer e.g. by conventional moulding, whereby the entire interior cavity is covered by a soft and pleasant material.

The invention will be explained in greater detail below, describing only an exemplary embodiment with reference to the drawing, in which
Fig. 1 is an oblique perspective view of a cup according to the invention of the side facing downwards,
Fig. 2 is a cross section of the cup shown in fig. 1.

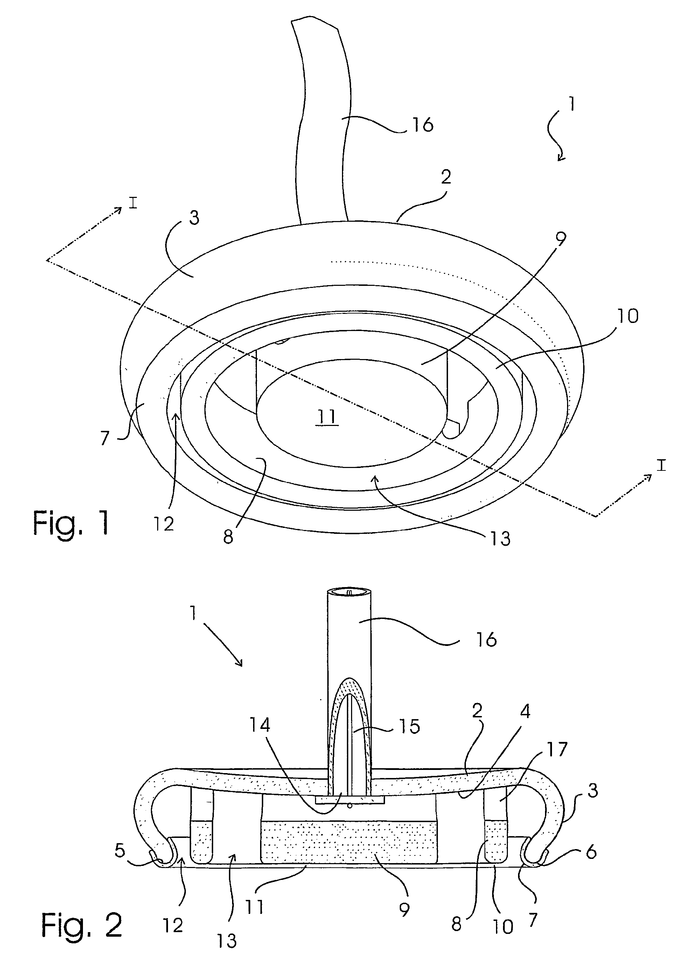

Fig. 1 shows an obstetrical vacuum cup according to the invention generally designated by the reference numeral 1. The cup is especially intended for being used in assisted delivery techniques connected to an external vacuum source. This external vacuum pump could e.g. be a conventional vacuum pump but it can also within the scope of the present invention be an integrated part of the device.

The vacuum cup 1 comprises a base 2, a sidewall 3, and a hollow interior cavity 4 for placement against the head of a fetus. The sidewall 3 has a distal edge 5 that provides a first contact surface 6. The edge 5 is covered with la sealing member 7, ensuring that the cup 1 makes an effectively seal against the fetal head. The sealing member is made of an elastomeric material, providing a relatively soft contact surface with the fetal head.

Inside, the interior cavity 4 is provided with a first collar-shaped projection 8 and a second cylinder-shaped projection 9 arranged perpendicular on the base 2. Said projections 8,9 are providing the second contact surface 10,11 with a fetal head as will be described in detail below.

The first and second projections 8,9 divide the interior cavity into two concentric vacuum compartments 12,13. The first compartment 12 is arranged between the annual sidewall 3 of the cup 1 and the first projection 8, whereas the second compartment 13 is arranged between the first 8 and second 9 projection.

As is evident from fig. 2 the base 2 is provided with a vacuum opening 14 communicating with the interior cavity 4 of the vacuum cup.

The vacuum cup 1 is further provided with an elongated traction device 15, such as a cord or a chain inside the vacuum tube, with a handle (not shown) for applying the traction force. An elongated tube 16 is at one end coupled to the vacuum opening 14 of the cup 1 and the other end to a hand-held pump (not shown).

The traction device 15 and the tube 16 are in this embodiment combined, providing the physician with the opportunity to apply the extraction force and manoeuvre the cup with the same hand.

To facilitate delivery, the vacuum cup 1 is applied to the fetal head so that the center of the cup corresponds as close as possible to the flexion point. In this position the head of the infant is only in contact with the sealing member 7 placed on the distal edge 5 of the cup, i.e. the distance from the base 2 to the first contact surface 6 is longer than the distance from the base 2 to the second surface 10,11.

Operation of the hand-held pump will then result in the development of a vacuum between the cup 1 and the infant's head. To better distribute the applied vacuum within the cup 1, the vacuum cup is provided with a vacuum channel 17 in each projection 8,9. Additionally, the diameter of the cup at the distal edge 5 of the cup 1 is slightly smaller than the diameter of the cup at the sidewall 3, as may be seen in fig. 2, such that the vacuum flow may also be distributed along the interior of the sidewall 2 of the cup 1.

Application of vacuum to the interior cavity 4 will bring the second contact surface 10,11 of the two projections 8,9 into contact with the fetal head providing both a physical barrier and providing an efficient vacuum seal. The physical barrier will reduce or even eliminate the risk that blood and/or body fluids from the mother and/or fetus will be drawn through the vacuum tube into the external vacuum source, and will thereby prevent a possible contamination of the vacuum source.

The plurality of contact surfaces significantly increase the surface area of the cup which contacts and forms the seal with the fetal head when vacuum is applied, ensuring that the traction forces resulting from the application of vacuum and manipulation are distributed over a greater area of the fetal head. Accordingly, the potential for injury to the fetus caused by a concentration of forces is reduced.

The projections 10,11 are made of a relatively hard electromeric material having memory. That is to say, the projections will automatically try to return to their original shape when they have been mechanically deformed and then relieved. The material will in this case only allow the scalp of the infant to be drawn into the two vacuum compartments 12,13, thereby significantly reducing the swelling of the scalp normally associated with vacuum extractors.

The vacuum compartments 12,13 further provide the advantage that if the first contact surface 6 detaches from the fetal scalp during extraction, only the first vacuum compartment 12, which is adjacent to the distal edge 5 of the cup 1, will loose its grip with the fetal scalp. The vacuum seal in the second vacuum compartment 13 will not break immediately. This will ensure, that the operator has sufficient time to either support the fetal head before the cup 1 detaches in full or even have an opportunity to reattach the cup by applying additional vacuum.

The jerks of the fetus and the lacerations on the scalp normally involved in cup detachments will also be reduced. Furthermore, since the sealing member 7 covers the distal edge 5, it will reduce the risk of lacerations further, especially if the cup 1 slides across the fetel scalp after a detachment.

The extractor cup according to the invention can be a disposable cup, i.e. a cup that is discarded after being used and is replaced each time by a new, sterile cup. These disposable cups are each provided with means, such as a junction, for connecting the cup to the external vacuum source, such as a hand held pump. The cup will therefore always be able to function effectively and hygienically as far as this component is concerned.

Furthermore, a sterile vacuum extractor can in e.g. stressed situation be restored quickly and easily without having to spend time and energy on subsequent sterilisation of the cup in this connection.

## Claims

1. An obstetrical vacuum cup (1) comprising a base (2) and a side wall (3) defining a hollow interior cavity (4), said side wall having a distal edge (5) which defines a first contact surface (6); and a vacuum opening (14) communicating with the interior cavity of the vacuum cup and being adapted for connection to an external vacuum source wherein the hollow interior cavity is designed with at least one projection (8,9) for providing at least one second contact surface (10,11) when vacuum is applied from said external source of vacuum, **characterized in, that** at least one vacuum channel (17) extends from the vacuum opening (14) through the at least one projection (8,9).

2. A vacuum cup according to claim 1, **characterized in, that** the at least one projection (8,9) is a concentric collar, ring or cylinder arranged on the interior of the base. (2).

3. A vacuum cup according to claim 1 or 2, **characterized in, that** the at least one projection (8,9) defines a number of concentric vacuum compartments (12,13).

4. A vacuum cup according to claim 3, **characterized in, that** a first vacuum compartment (12) is arranged between the side wall (3) of the cup and a first projection (8), and a second vacuum compartment (13) is arranged between the first (8) and a second projection (9).

5. A vacuum cup according to claim 1 - 4, **characterized in, that** the projection (8,9) is made of an elastomeric material.

6. A vacuum cup according to claim 1 - 5, **characterized in, that** the edge of the side wall (3) is at least partly covered with a sealing member (7).

7. A vacuum cup according to claim 6, **characterized in, that** the sealing member (7) is made of a relatively soft elastomeric material.

8. A vacuum cup according to any of claims 1-7, **characterized in, that** the cup (1) further comprises at least one annular ridge extending about the periphery of the distal edge of the sidewall (3).

## Patentansprüche

1. Saugglocke für die Geburtshilfe mit einer Basis (2) und einer Seitenwandung (3), die einen hohlen Innenhohlraum (4) bilden, wobei die Seitenwandung eine distale Kante (5) hat, die eine erste Kontaktfläche (6) bildet; und einer Vakuumöffnung (14), die mit dem Innenhohlraum der Saugglocke kommuniziert und die zur Verbindung mit einer externen Vakuumquelle eingerichtet ist, wobei der hohle Innenhohlraum mit wenigstens einer Ausbuchtung (8, 9) zum Bereitstellen wenigstens einer zweiten Kontaktfläche (10, 11) konstruiert ist, wenn von der externen Vakuumquelle Vakuum angelegt ist,
**dadurch gekennzeichnet, dass**
sich wenigstens ein Vakuumkanal (17) von der Vakuumöffnung (17) durch die wenigstens eine Ausbuchtung (8, 9) erstreckt.

2. Saugglocke nach Anspruch 1, **dadurch gekennzeichnet, dass** die wenigstens eine Ausbuchtung (8, 9) ein konzentrischer Kragen, ein Ring oder ein Zylinder ist, der am Inneren der Basis (2) angeordnet ist.

3. Saugglocke nach Anspruch 1 oder 2, **dadurch gekennzeichnet, dass** die wenigstens eine Ausbuchtung (8, 9) eine Anzahl von konzentrischen Vakuumkompartimenten (12, 13) bildet.

4. Saugglocke nach Anspruch 3, **dadurch gekennzeichnet, dass** ein erstes Vakuumkompartiment (12) zwischen der Seitenwandung (3) der Glocke und einer ersten Ausbuchtung (8), und ein zweites Vakuumkompartiment (13) zwischen der ersten (8) und einer zweiten Ausbuchtung (9) angeordnet ist.

5. Saugglocke nach Anspruch 1 - 4, **dadurch gekennzeichnet, dass** die Ausbuchtung (8, 9) aus einem elastomeren Material hergestellt ist.

6. Saugglocke nach Anspruch 1 - 5, **dadurch gekennzeichnet, dass** die Kante der Seitenwandung (3) wenigstens teilweise von einer Manschette (7) bedeckt ist.

7. Saugglocke nach Anspruch 6, **dadurch gekennzeichnet, dass** die Manschette (7) aus einem relativ weichen elastomeren Material hergestellt ist.

8. Saugglocke nach einem der Ansprüche 1 - 7, **dadurch gekennzeichnet, dass** die Saugglocke (1) weiter wenigstens eine ringförmige Leiste aufweist, die sich über den Rand der distalen Kante der Seitenwandung (3) erstreckt.

## Revendications

1. Ventouse obstétrique (1) comprenant une base (2) et une paroi latérale (3) définissant une cavité intérieure creuse (4), ladite paroi latérale ayant un bord distal (5) qui définit une première surface de contact (6) ; et une ouverture (14) destinée à établir une dépression, l'ouverture communiquant avec la cavité intérieure de la ventouse et étant adaptée pour être raccordée à une source externe permettant de créer une dépression, dans laquelle la cavité intérieure creuse est conçue avec au moins une saillie (8,9) destinée à fournir au moins une deuxième surface de contact (10, 11) lorsque la dépression est appliquée depuis la source externe, **caractérisée en ce qu'**au moins un canal de dépression (17) s'étend depuis l'ouverture (14) destiné à établir une dépression à travers la ou chaque projection (8,9).

2. Ventouse selon la revendication 1, **caractérisée en ce que** la ou chaque saillie (8,9) est un collier, anneau ou cylindre agencé sur l'intérieur de la base (2).

3. Ventouse selon la revendication 1 ou 2, **caractérisée en ce que** la ou chaque saillie (8,9) définit un certain nombre de compartiments (12, 13) à dépression concentriques.

4. Ventouse selon la revendication 3, **caractérisée en ce que** un premier compartiment à dépression (12) est agencé entre la paroi latérale (3) de la ventouse et une première saillie (8), et un deuxième compartiment à dépression (13) est agencé entre la première (8) et une deuxième (9) saillies.

5. Ventouse selon l'une quelconque des revendications 1 à 4, **caractérisée en ce que** la saillie (8,9) est fabriquée dans un matériau élastomère.

6. Ventouse selon l'une quelconque des revendications 1 à 5, **caractérisée en ce que** le bord de la paroi latérale (3) est au moins partiellement recouvert avec un élément d'étanchéité (7).

7. Ventouse selon la revendication 6, **caractérisée en ce que** l'élément d'étanchéité (7) est fabriqué dans un matériau élastomère relativement mou.

8. Ventouse selon l'une quelconque des revendications 1 à 7, **caractérisée en ce que** la ventouse (1) comprend en outre au moins une nervure annulaire s'étendant sur la périphérie du bord distal de la paroi latérale (3).
